# EUROPEAN PATENT SPECIFICATION

(11) **EP 2 617 367 B1**
(45) Date of publication and mention of the grant of the patent: **23.03.2016**
(21) Application number: 12792474.4
(22) Date of filing: 31.05.2012
(51) Int. Cl.: A61B 17/04

(54) **SUTURE THREAD PUSHING APPARATUS AND SUTURE THREAD PUSHING SYSTEM**
NAHTFADENSCHUBVORRICHTUNG UND NAHTFADENSCHUBSYSTEM
DISPOSITIF DE POUSSÉE D'UNE SUTURE ET SYSTÈME DE POUSSÉE D'UNE SUTURE

(30) Priority: 02.06.2011 US 201161492494 P
(43) Date of publication of application: 24.07.2013
(73) Proprietor: Olympus Corporation, Shibuya-ku Tokyo (JP)
(72) Inventor: TAKAHASHI Shinji, Tokyo 151-0072 (JP)
(74) Representative: Schicker, Silvia
(86) International application number: PCT/JP2012/064084
(87) International publication number: WO 2012/165552

(56) References cited:
- EP-A1- 1 952 770
- JP-A- 5 192 338
- JP-A- 9 266 910
- JP-A- H08 501 001
- JP-A- 2007 500 575
- JP-A- 2010 506 663
- US-A- 5 163 946
- US-A- 6 132 439
- US-A1- 2005 033 319
- US-A1- 2006 161 183
- US-A1- 2010 113 873
- US-A1- 2010 145 364

## Description

### Technical Field of the Invention

The present invention relates to a suture thread pushing apparatus that moves a twist portion that is formed in a suture thread, and to a suture thread pushing system.

### Background Art

Conventionally, in a surgical operation and the like, suture thread is used for the suture of tissue. In order to maintain the state in which the tissue has been sutured, a knot (a connection portion) is formed in the suture thread by twisting the two end portions of the suture thread around each other twice so that the suture thread is tied.

For example, the suture thread pushing apparatus described in Patent Literature 1 is provided with an elongated shaft, and a first holding member and a second holding member that are mounted at a distal end of the elongated shaft. Overall, the suture thread pushing apparatus is formed in a two-pronged shape. Opening portions (side holes) are formed in a central portion in a longitudinal direction of the respective holding members, and recessed portions are formed in a distal ends of the respective holding members.

After an operator makes the suture thread pass through target tissue, the suture thread is extended to an outside of a patient, and a knot is formed therein. The knot is laid between the first holding member and the second holding member, and one end portion of the suture thread is engaged with the recessed portion of the first holding member, and is inserted through the opening portion via an outer surface of the first holding member. The one end portion of the suture thread is then guided from between the two holding members to a proximal side of the elongated shaft. In the same way, the other end portion of the suture thread is also engaged with the recessed portion of the second holding member, and is inserted through the opening portion via an outer surface of the second holding member. The other end portion of the suture thread is then guided from between the two holding members to the proximal end side of the elongated shaft.

When the operator moves the suture thread pushing apparatus toward the tissue while holding the two end portions of the suture thread, the suture thread forming the knot kept in between the two end portions is moved towards the proximal side relative to the suture thread pushing apparatus, so that the knot is moved toward the tissue.

However, in the above-described suture thread pushing apparatus, there is an issue that the tissue cannot be ligated in a state in which the tissue is bound tightly with the suture thread, because the knot cannot be attached firmly to the tissue and a gap between the knot and the tissue is created when the suture thread is fine relative to a recess in the recessed portion.

A suture thread pushing apparatus resolving this issue is disclosed in FIGS. 56 to 58 of Patent Literature 2. In this suture thread pushing apparatus, slits are formed in a tubular member (an outer cylinder) mounted at the distal end thereof, and a suture thread capturing mechanism (a rod-shaped object) is able to advance and retreat inside the tubular member. The knot in the suture thread is arranged so as to face an opening in the tubular member, and the one end portion and the other end portion of the suture thread are inserted respectively through the slits from an inside of the tubular member toward an outside of the tubular member.

In this state, since an operator moves the suture thread capturing mechanism toward a distal side while holding the two ends of the suture thread, the knot can be tightened.

Moreover, in recent years, soft suture thread pushing apparatuses having flexibility have become widely used in order to deal with shapes of human digestive tract which bends in complex directions, or the like.

### Citation List

### Patent Literature

Patent Literature 1: Japanese Unexamined Patent Application, First Publication No. 2007-244867
Patent Literature 2: Published Japanese Translation No. 2008-500125 of the PCT International Publication

US 5,163,946 discloses a knot rundown tool which comprises an elongate shaft having an axial bore coupled with a front end of the shaft. The shaft also includes a groove in the front end thereof for receiving a suture throw. The shaft also includes a slot extending through a central axis of the shaft perpendicular to the long axis of the shaft. The slot is spaced rearwardly from the front groove. The tool also includes a sleeve which surrounds the front end of the shaft and is mounted for axial, reciprocal movement relative to the front end of the shaft. The sleeve has a hollow interior sized so that the shaft may be received therein with a free axially-sliding fit. The sleeve includes a slot which extends through and perpendicular to the longitudinal axis of the sleeve and is coupled with the front end of the sleeve. Axial movement of the sleeve relative to the front end of the shaft is limited by a pin which is attached to the sleeve so as to extend through the hollow interior of the sleeve perpendicular to the long axis of the sleeve. The sleeve is positioned relative to the shaft so that the pin is received in a slot in the shaft. As the sleeve is moved axially relative to the shaft, the pin will contact the top and bottom ends of the slot, thereby preventing further movement of the sleeve relative to the shaft.

### Summary of the Invention

### Problems to be Solved by the Invention

However, if flexible members are applied to the suture thread pushing apparatus disclosed in Patent Literature 2, the suture thread pushing apparatus is easy to be twisted because stiffness of the suture thread capturing mechanism in the suture thread pushing apparatus is decreased. Therefore, there is a possibility that a position of the tubular member in a circumferential direction thereof shifts relative to a position of the suture thread capturing mechanism in that direction.

The present invention is developed in view of the above-described situation, and the purpose of the present invention is to provide a suture thread pushing apparatus which can prevent a position of the tubular member in a circumferential direction thereof from shifting relative to a position of the suture thread capturing mechanism in that direction, and which can prevent a suture thread from becoming jammed.

### Means for Solving the Problem

This problem is addressed by a suture thread apparatus according to claim 1. A suture thread pushing apparatus may include: an outer cylinder that has flexibility; and a rod-shaped object that has flexibility and is inserted into the outer cylinder so as to advance and retreat, wherein a pair of side holes that face each other is formed in a distal side of the outer cylinder such that the suture thread passes through the side holes, an engaging portion that extends in an axial direction of the outer cylinder is formed in an inner surface on the distal side of the outer cylinder, and an engaged portion that engages with the engaging portion and is capable of sliding along the engaging portion is formed on an outer surface of the rod-shaped object.

Moreover, in the suture thread pushing apparatus, the engaging portion may be a slit formed in the outer cylinder, and the engaged portion may be a projection protruding from an outer surface of the rod-shaped object.

Moreover, in the suture thread pushing apparatus, spaces may be formed between an outer surface of a distal end portion of the rod-shaped object and an inner surface of the outer cylinder such that the suture thread is kept in the spaces when the rod-shaped object is inserted into the outer cylinder.

Moreover, in the suture thread pushing apparatus, the rod-shaped object may be formed longer than the outer cylinder.

Moreover, in the suture thread pushing apparatus, an outer surface of a distal end portion of the rod-shaped object may include: a first distal end side surface that extends as far as a distal end surface of the rod-shaped object; and a second distal end side surface that is arranged on an opposite side of the rod-shaped object from the first thread catch surface, is formed substantially parallel to the first distal end side surface, and extends as far as the distal end surface of the rod-shaped object.

Moreover, in the suture thread pushing apparatus, a groove portion may be formed in a distal end surface of the rod-shaped object, and both ends of the groove portion may extend to edge portions of the distal end surface.

Moreover, in the suture thread pushing apparatus, a pair of grooves that extends from a distal end surface of the rod-shaped object and is arranged in opposite positions on both side of the rod-shaped object may be formed in the outer surface of the rod-shaped object, and each of the grooves may be formed in a substantially semicircular shape in a direction parallel to an axial direction of the rod-shaped object.

Moreover, a suture thread pushing system may include the suture thread pushing apparatus, and an endoscope including an insertion portion configured to observe a distal side of the insertion portion.

Moreover, the suture thread pushing system may further include a guide tube having flexibility, wherein the outer cylinder is inserted into a channel of the guide tube so as to advance and retreat, and the guide tube is arranged in parallel with the insertion portion and is fixed onto an outer surface of the insertion portion.

Moreover, in the suture thread pushing system, a channel into which the outer cylinder is inserted so as to advance and retreat may be formed in the insertion portion.

Moreover, in the suture thread pushing system, the insertion portion may be inserted into a channel of the outer cylinder so as to advance and retreat.

Moreover, in the suture thread pushing system, the insertion portion may be flexible, and a channel through which the rod-shaped object is inserted so as to advance and retreat may be formed in the insertion portion.

Moreover, in the suture thread pushing system, the outer cylinder may be configured to be capable of being fixed to a distal end of the insertion portion.

Moreover, in the suture thread pushing system, the insertion portion may be flexible, and a channel through which the rod-shaped object is inserted so as to advance and retreat may be formed in the insertion portion.

Moreover, in the suture thread pushing system, the rod-shaped object may be formed longer than the channel in the insertion portion.

Moreover, in the suture thread pushing system, an outer surface of a distal end portion of the rod-shaped object which the suture thread pushing apparatus includes may include: a first distal end side surface that extends as far as a distal end surface of the rod-shaped object; and a second distal end side surface that is arranged on an opposite side of the rod-shaped object from the first distal end side surface, is formed substantially parallel to the first distal end side surface, and extends as far as the distal end surface of the rod-shaped object.

Moreover, in the suture thread pushing system, a groove portion whose two ends extend to edge portions of a distal end surface of the rod-shaped object may be formed in the distal end surface.

### Effects of the Invention

According to the above-described suture thread pushing apparatus, it is possible to prevent orientations of the distal end of the shaft from shifting in the circumferential direction relative to the outer cylinder.

### Brief Description of the Drawings

FIG. 1 is a perspective view of a suture thread pushing apparatus of the present invention.
FIG. 2 is a cross-sectional plan view of the suture thread pushing apparatus of the present invention.
FIG. 3 is a cross-sectional view illustrating a suture thread pushing method used for the suture thread pushing apparatus of the present invention.
FIG. 4 is a cross-sectional view illustrating the suture thread pushing method used for the suture thread pushing apparatus of the present invention.
FIG. 5 is a cross-sectional view illustrating the suture thread pushing method used for the suture thread pushing apparatus of the present invention.
FIG. 6 is an overall view of a suture thread pushing system of the present invention.
FIG. 7 is an overall view of further a suture thread pushing system of the present invention.
FIG. 8 is a perspective view showing a principal portion of a modified suture thread pushing system of the present invention.
FIG. 9 is a partially cutaway perspective view showing a principal portion of a modified suture thread pushing system of the present invention.
FIG. 10 is a partially cutaway perspective view showing a principal portion of a modified suture thread pushing system of the present invention.
FIG. 11 is a perspective view of a shaft in a suture thread pushing apparatus.
FIG. 12 is a perspective view of a shaft in a suture thread pushing apparatus.
FIG. 13 is a perspective view of a shaft in a suture thread pushing apparatus.
FIG. 14 is a perspective view of a shaft in a suture thread pushing apparatus.
FIG. 15 is an explanatory view of a grasping forceps used for a suture thread pushing apparatus.

### Embodiments of the Invention

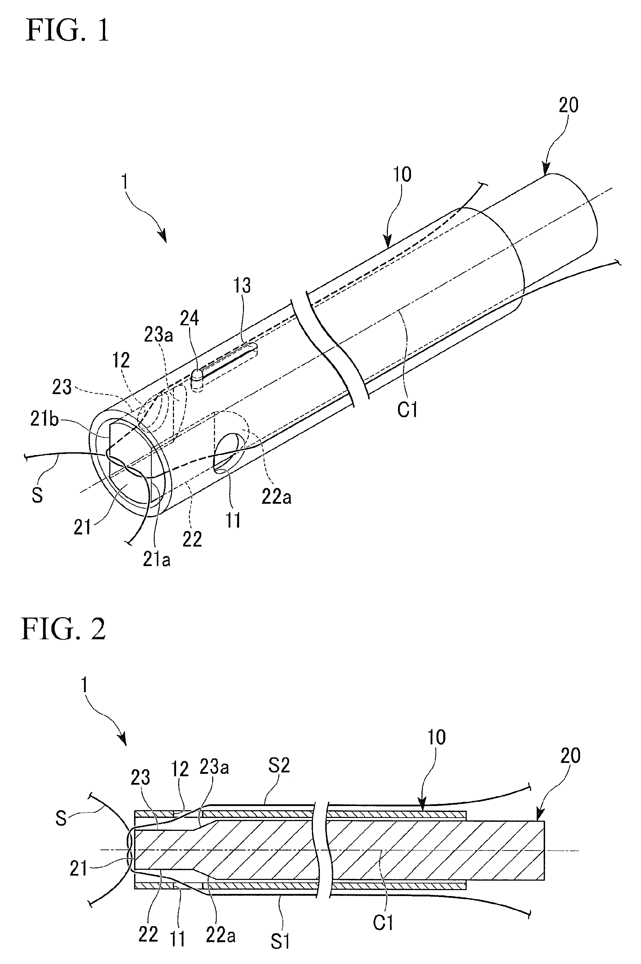

Hereinafter, a suture thread pushing apparatus according to the present invention is described referring to FIG. 1 through FIG. 5. This suture thread pushing apparatus (hereinafter "the pushing apparatus") is used to tie up a tissue in a human body with a suture thread inserted through the tissue. Namely, two end portions of a suture thread are guided to the outside of the body via a natural orifice, and the pushing apparatus moves a twist portion (a connecting portion) that is formed by the two end portions to a sutured portion of the tissue.

As shown in FIG. 1 and FIG. 2, the pushing apparatus 1 is provided with a flexible outer cylinder 10 and a shaft (a rod-shaped object) 20 that is flexible and is inserted into the outer cylinder 10 so as to be able to advance and retreat.

A pair of side holes 11 and 12 is formed facing each other in a distal side of the outer cylinder 10 such that a suture thread S is able to be inserted through them. Note that it is preferable that the side holes 11 and 12 are formed adjacent to a distal end of the outer cylinder 10.

A slit (an engaging portion) 13 extending in the direction of an axis C1 of the outer cylinder 10 is formed in the distal side of the outer cylinder 10 (see FIG. 1). For instance, the outer diameter of the outer cylinder 10 is set to approximately 5 to 10 millimeters, and the length of the outer cylinder 10 is set to approximately several hundred millimeters. The outer cylinder 10 is made of a biocompatible flexible material such as silicone, PTFE, rubber, and superelastic alloy like Ni-Ti.

The shaft 20 is formed in an elongated cylindrical shape. The shaft 20 is longer than the outer cylinder 10 in the direction of the axis C1.

A first distal end side surface 22 and a second distal end side surface 23 that extend as far as a distal end surface 21 of the shaft 20 are formed on an outer surface of a distal end portion of the shaft 20. The first distal end side surface 22 is formed so as to be substantially parallel to the axis C1. The second distal end side surface 23 is arranged on the opposite side of the shaft 20 from the first distal end side surface 22, and is formed so as to be substantially parallel to the first distal end side surface 22.

A thread catch portion 21a is formed by a connection portion where the first distal end side surface 22 and the distal end surface 21 are connected each other, and a thread catch portion 21b is formed by a connection portion where the second distal end side surface 23 and the distal end surface 21 are connected each other. The thread catch portion 21a and the thread catch portion 21b are arranged so as to be substantially parallel to each other.

The first distal end side surface 22 is configured to form a space between the first distal end side surface 22 and an inner surface of the outer cylinder 10 such that the suture thread S is able to be kept in the space when the shaft 20 is inserted into the outer cylinder 10. A proximal end of the first distal end side surface 22 is connected to a tapered surface 22a that is gradually separated from the axis C1 as the proximal end of the tapered surface 22a approaches. The second distal end side surface 23 is configured in the same way as the first distal end side surface 22, namely, so as to form a space. A proximal end of the first distal end side surface 22 is connected to a tapered surface 23a that is formed so as to be axisymmetric to the tapered surface 22a about the axis C1.

The width of the distal end surface 21 is formed narrower than the outer diameter of the shaft 20 due to the distal end side surfaces 22 and 23 of the shaft 20. Because of this, when the shaft 20 is inserted into the outer cylinder 10, the space formed by the inner surface of the outer cylinder 10 and the distal end side surfaces 22 and 23 of the shaft 20 is larger than a gap between the inner surface of the outer cylinder 10 and the outer surface of the shaft 20 except for portions of the shaft 20 having the distal end surfaces 22 and 23 and the tapered surfaces 22a and 23b.

Note that the gap between the inner surface of the outer cylinder 10 and the outer surface of the shaft 20 except for the portions of the shaft 20 having the distal end side surfaces 22 and 23 and the tapered surfaces 22a and 23a is set smaller than the outer diameter of the suture thread S.

A projection (an engaged portion) 24, which engages with the slit 13 and is able to slide in the direction of the axis C1 inside the slit 13, is formed on the outer surface of the shaft 20 (see FIG. 1).

The length of the aforementioned slit 13 in the direction of the axis C1 is set such that the pushing apparatus 1 reaches a pushing state when the projection 24 engages with a distal end of the slit 13. Here, the pushing state is a state in which the distal end surface 21 of the shaft 20 coincides with the distal end of the outer cylinder 10 as shown in FIG. 1 and FIG. 2, or a state in which the shaft 20 protrudes by approximately 1 ∼ 2 mm on a distal side from the outer cylinder 10. In the same way, the length of the slit 13 in the direction of the axis C1 is set such that the pushing apparatus 1 reaches a standby state in which the distal end surface 21 is located closer to a proximal side of the pushing apparatus 1 than the side holes 11 and 12, when the projection 24 engages with a proximal end of the slit 13.

The shaft 20 is made of rubber, resin, metal (including superelastic alloy), or the like that has elasticity. Metal coils can be favorably used as the metal having elasticity.

Next, a suture thread pushing method used for the pushing apparatus 1 having the above described structure is described. This suture thread pushing method includes: a winding step in which the suture thread S is wound around the pushing apparatus 1; a contact step in which the distal end of the outer cylinder 10 is brought into contact with a tissue; and a moving step in which the shaft 20 is moved towards a distal side of the pushing apparatus 1 such that the pushing apparatus 1 reaches the pushing state.

Note that in the following description, a case in which an aperture formed in a stomach wall is sutured is described, but the location to be treated is not limited to this and hollow organs such as, for example, an esophagus, a duodenum, a small intestine, a large intestine, an uterus, and a bladder may also be treated. Moreover, a natural orifice through which the pushing apparatus 1 is inserted is not limited to a mouth and may also be a nose or an anus.

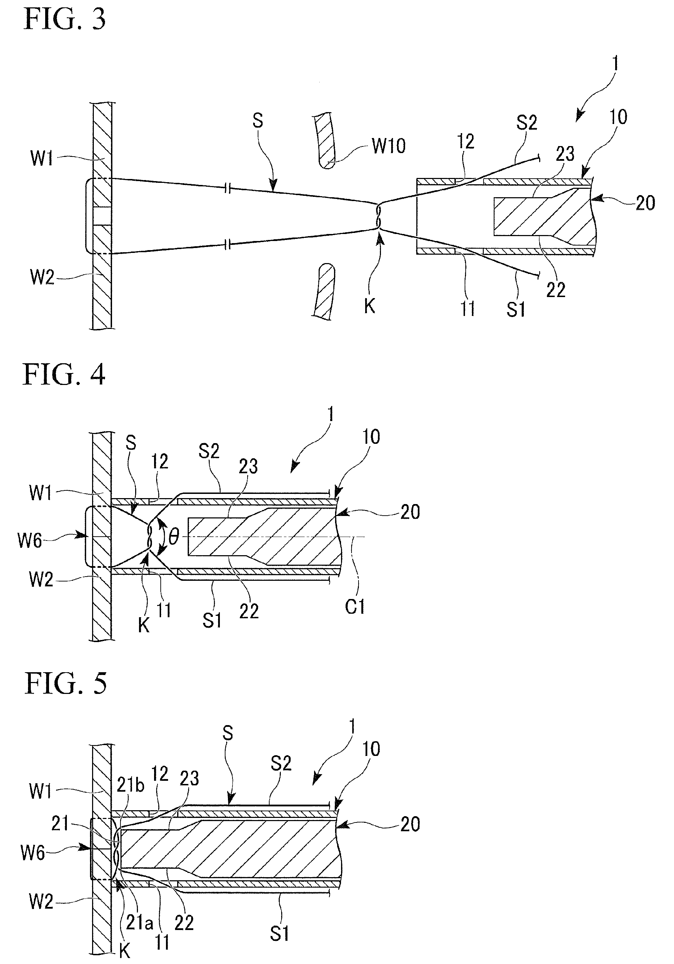

An operator inserts a flexible endoscope and a treatment tool through the mouth of a patient and into the interior of the stomach. The operator then makes an incision in the stomach wall while observing the operation by the endoscope, and removes the object to be treated. As shown in FIG. 3, the operator sews the tissue W1 and W2 on both sides of the incised portion with the suture thread S using a suture needle and needle forceps (not shown). The operator draws both end portions S1 and S2 of the suture thread S to the outside of the body through the mouth W10, and then twists the one end portion S1 around the other end portion S2 so as to form a twist portion K.

In the winding step, the operator pulls the shaft 20 toward the proximal side relative to the outer cylinder 10, so as to set the pushing apparatus 1 in the standby state. The operator inserts the one end portion S1 of the suture thread S into a channel in the outer cylinder 10 from the distal end of the outer cylinder 10, and then guides the one end portion S1 to the outside of the outer cylinder 10 through the side hole 11. In addition, the operator inserts the other end portion S2 of the suture thread S into the channel in the outer cylinder 10 from the distal end of the outer cylinder 10, and then guides the other end portion S2 to the outside of the outer cylinder 10 through the side hole 12.

Next, in the contact step, as shown in FIG. 4, the operator pushes the pushing apparatus 1 while holding the two end portions S1 and S2 of the suture thread S, and thereby brings the distal end of the outer cylinder 10 into contact with a suture portion W6 of the tissue W1 and W2 through the mouth W10. Because the distal end of the outer cylinder 10 and the side holes 11 and 12 are separated from each other by some distance in the direction of the axis C1 and the operator is holding the end portions S1 and S2, the twist portion K is not in close contact with the tissue W1 and W2.

Because the side holes 11 and 12 are formed facing each other on the outer cylinder 10, a thread crossing angle θ formed by the one end portion S1 and the other end portion S2 on the proximal side of the twist portion K is closer to 180° compared with the angle formed when the pair of side holes are not formed facing each other. Accordingly, the frictional resistance between the two suture threads S is reduced, and, furthermore, force used to push the pushing apparatus 1 can be effectively transmitted to tensile force of the suture thread S. As a consequence, the operator is able to smoothly push the pushing apparatus 1.

Next, in the moving step, as shown in FIG. 5, the operator pushes the shaft 20 toward the distal side relative to the outer cylinder 10, so that the pushing apparatus 1 reaches the pushing state. As a result, the twist portion K is moved toward the distal side of the outer cylinder 10 by the distal end surface 21 of the shaft 20. At this time, while holding the two end portions S1 and S2 of the suture thread S, the operator moves the twist portion K toward the distal side while bringing the two thread catch portions 21a and 21b of the shaft 20 into contact with the suture thread S.

Because the slit 13 in the outer cylinder 10 and the projection 24 on the shaft 20 are mutually engaged, even if the shaft 20 is moved backwards or forwards, it is possible to prevent the shaft 20 from rotating in a circumferential direction relative to the distal end of the outer cylinder 10.

The one end portion S1 of the suture thread S which is on the proximal side of the twist portion K is kept in the space between the first distal end side surface 22 and the inner surface of the outer cylinder 10, and is guided to the outside of the outer cylinder 10 through the side hole 11. In the same way, the other end portion S2 of the suture thread S which is on the proximal side of the twist portion K is kept in the space between the second distal end side surface 23 and the inner surface of the outer cylinder 10, and is guided to the outside of the outer cylinder 10 through the side hole 12.

The twist portion K then becomes in close contact with the tissue W1 and the tissue W2, so that the tissue W1 and the tissue W2 are tightly bound together.

Here, while the pushing apparatus 1 is in the standby state, the tissue W1 and the tissue W2 are held in their tightly bound state by the friction between the suture threads S. The operator removes the pushing apparatus 1 from the suture thread S, and then once again forms the twist portion K in the suture thread S outside the patient's body. The operator performs the above-described winding step, contact step, and moving step, and then two twist portions K are formed adjacent to each other in the suture thread S, so that a knot is formed.

The operator uses scissors to make a cut in a suitable location of the suture thread S, and takes the endoscope and the treatment tool out from the body of the patient through the mouth W10. The operation is thereby completed.

If flexible members are applied to the suture thread pushing apparatus disclosed in Patent Literature 2, the suture thread capturing mechanism is easy to be twisted due to decrease of stiffness, and therefore there is an issue that a position of the tubular member in a circumferential direction thereof shifts relative to a position of the suture thread capturing mechanism in that direction.

On the other hand, according to the pushing apparatus 1, the end portions S1 and S2 on the proximal side of the twist portion K in the suture thread S pass through the side holes 11 and 12 in the outer cylinder 10. In addition, since the operator pushes the pushing apparatus 1 while holding the end portions S1 and S2, the distal end of the outer cylinder 10 is brought into contact with the suture portion W6, and the twist portion K is moved to the distal side of the outer cylinder 10 by the distal end surface 21 of the shaft 20.

In addition, since the two mutually adjacent twist portions K in the suture thread S are formed into a knot while the suture portion W6 is kept firmly bound by means of the suture thread S, the tissue W1 and the tissue W2 can be ligated while being tightly bound together.

Furthermore, the slit 13 is formed in the outer cylinder 10, the projection 24 is formed on the shaft 20, and the projection 24 engages with the slit 13. Accordingly, it is possible to prevent orientations of the distal end side surfaces 22 and 23 of the shaft 20 from shifting in the circumferential direction relative to the outer cylinder 10.

Because the engaging portion is the slit 13 penetrating the outer cylinder 10 and the engaged portion is the projection 24, both the engaging portion and the engaged portion can be formed easily.

Because the distal end side surfaces 22 and 23 are formed on the shaft 20 and spaces are formed between these and the inner surface of the outer cylinder 10, it is possible to prevent the suture thread S from becoming jammed between the outer surface of the shaft 20 and the inner surface of the outer cylinder 10.

Because the shaft 20 is formed longer than the outer cylinder 10, the shaft 20 protrudes from the proximal end of the outer cylinder 10 regardless of whether the pushing apparatus 1 is in the pushing state or the standby state. As a consequence, the operator is able to easily manipulate the shaft 20 from the proximal side of the outer cylinder 10.

Because the thread catch portions 21a and 21b are formed on the shaft 20 by the distal end side surfaces 22 and 23 and the distal end surface 21, it is possible to prevent the position of the suture thread S from shifting from the distal end surface 21 of the shaft 20 by the thread catch portions 21a and 21b being brought into contact with the suture thread S.

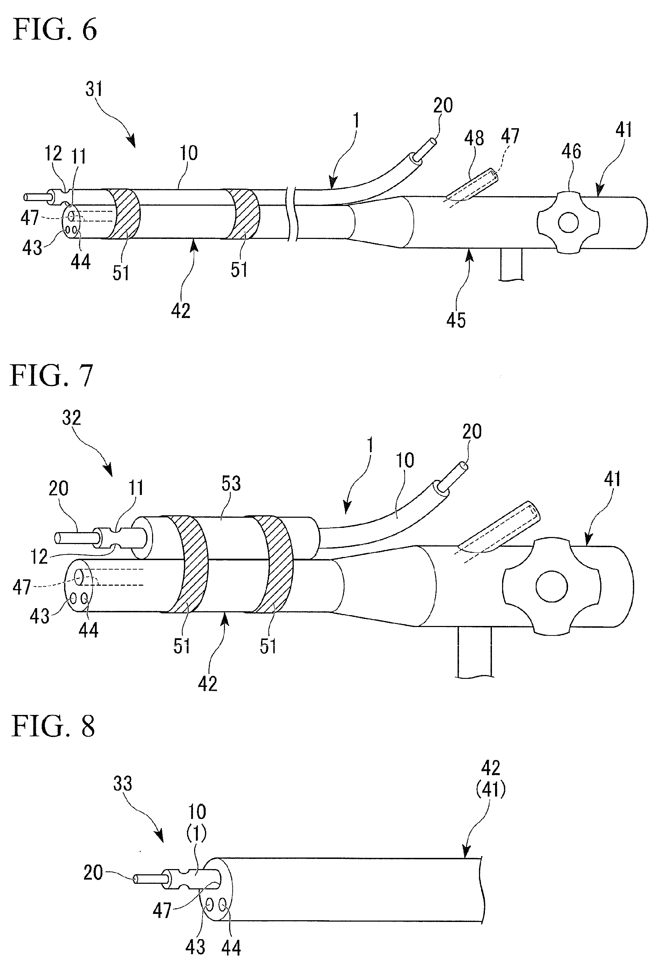

Next, a suture thread pushing system of the present invention is described referring to FIG. 6 through FIG. 10. Note that portions that are the same as in the above-described suture thread pushing apparatus are given the same descriptive symbols and any description thereof is omitted. Only points different from the foregoing apparatus are described. A suture thread pushing system of the present invention is composed of the above-described pushing apparatus and an endoscope. Hereinafter, this suture thread pushing system is described.

As shown in FIG. 6, a suture thread pushing system (hereinafter, "the pushing system") 31 is provided with the above-described pushing apparatus 1 and an endoscope 41 having an insertion portion 42 that is able to observe the distal side thereof.

A light-emitting unit 43 and a light-receiving unit 44 are mounted on the distal end surface of the insertion portion 42. Observation light emitted from the light-emitting unit 43 is reflected by a test object, and then the reflected light is detected by the light-receiving unit 44. As a result, an operator is able to observe a condition in front of the distal end of the insertion portion 42. A manipulation portion 45 is connected to a proximal end of the insertion portion 42. By operating a knob 46 mounted on the manipulation portion 45, the operator can bend a bending portion that is mounted on the distal side of the insertion portion 42.

A channel 47 is formed in the insertion portion 42. A distal end of the channel 47 opens onto the distal end surface of the insertion portion 42, and a proximal end of the channel 47 communicates with a forceps opening 48 mounted on the manipulation portion 45.

The outer cylinder 10 of the pushing apparatus 1 and the insertion portion 42 of the endoscope 41 are arranged in parallel and next to each other, and are fixed together by medical fixing bands 51 or the like.

According to the pushing system 31 configured in the above described manner, the ligating of the suture thread S can be performed more reliably because the operator can constantly observe the step of pushing the twist portion K by using the pushing apparatus 1 with the endoscope 41.

The structure of the pushing system can be modified in various ways as described below.

For example, as a pushing system 32 shown in FIG. 7, the pushing system may include a guide tube 53 that can be attached to an outer surface of the insertion portion 42 with the guide tube 53 arranged in parallel with the insertion portion 42.

The guide tube 53 can be made of the same materials having flexibility as the outer cylinder 10. The inner diameter of the guide tube 53 is set larger than the outer diameter of the outer cylinder 10, and the outer cylinder 10 is inserted into a channel of the guide tube 53 so as to be able to advance and retreat.

The guide tube 53 and the insertion portion 42 are arranged in parallel and next to each other, and are fixed together by the aforementioned fixing bands 51 or the like.

In the pushing system 32 configured in the above described manner, it is possible to obtain the similar effects obtained from the pushing system 31.

Moreover, as a pushing system 33 shown in FIG. 8, the outer cylinder 10 may be configured to be inserted into the channel 47 of the endoscope 41 so as to be able to advance and retreat. In this modified pushing system, the outer diameter of the outer cylinder 10 is set smaller than the inner diameter of the channel 47.

In the pushing system 33 configured in this manner, it is also possible to obtain the similar effects obtained from the pushing system 31.

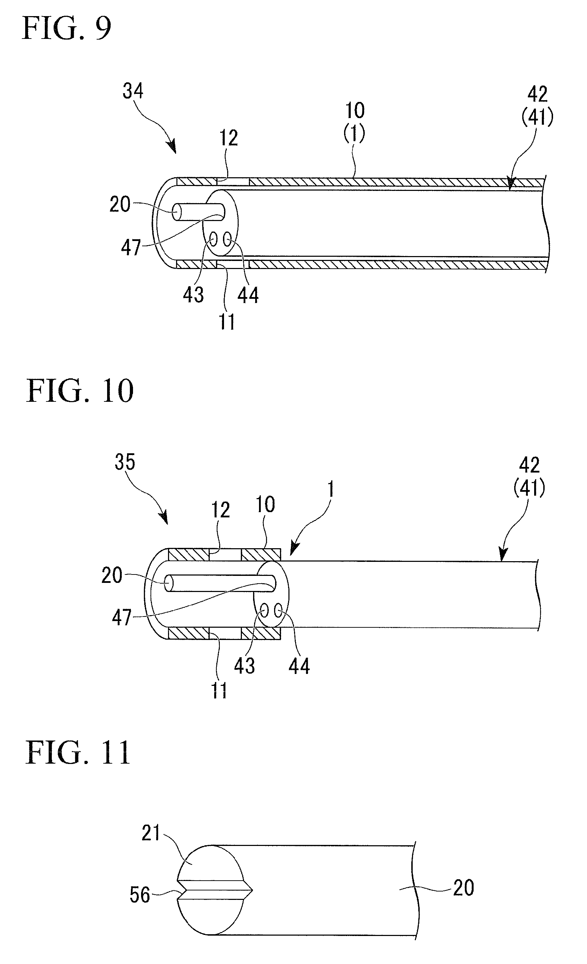

As a pushing system 34 shown in FIG. 9, the insertion portion 42 of the endoscope 41 may be configured to be inserted into the channel of the outer cylinder 10 so as to be able to advance and retreat. In this modified pushing system, the outer diameter of the insertion portion 42 is set smaller than the inner diameter of the outer cylinder 10. This configuration can be preferably used in a case in which the outer cylinder 10 has a large outer diameter like an overtube.

Moreover, in this modified pushing system, the insertion portion 42 may be made of a flexible material such as a coil or the like, and the shaft 20 may be configured to be inserted into the channel 47 of the endoscope 41 so as to be able to advance and retreat. In this configuration, because the insertion portion 42, the outer cylinder 10, and the shaft 20 are all made of flexible materials, it is easy to bend the insertion portion 42, the outer cylinder 10, and the shaft 20 as an integrated body.

Moreover, in this modified pushing system, the shaft 20 is preferably set longer than the channel 47. In this configuration, the shaft 20 can be easily manipulated from a side of the forceps opening 48.

As a pushing system 35 shown in FIG. 10, the outer cylinder 10 may be configured to be able to be fixed to the distal end of the insertion portion 42. Mechanical engagement, press fitting, connection by an adhesive, or the like can be used to fix the insertion portion 42 and the outer cylinder 10 together. The outer cylinder 10 is fixed onto the insertion portion 42 like a cap. In this configuration, the size of the outer cylinder 10 can be reduced.

Moreover, in this modified pushing system, the insertion portion 42 may be made of a flexible material such as a coil or the like, and the shaft 20 may be configured to be inserted into the channel 47 of the endoscope 41 so as to be able to advance and retreat. In this configuration, the insertion portion 42, the outer cylinder 10, and the shaft 20 are all made of flexible materials, and can be easily bent as a single integrated body.

Moreover, in this modified pushing system, the shaft 20 is preferably set longer than the channel 47. In this configuration, the shaft 20 can be easily manipulated from the side of the forceps aperture 48.

While the present invention has been described above referring to the drawings, the specific structure of the present invention is not limited to the described embodiments. Various modifications and the like may be made to the present invention without departing from the scope of the appended claims. Furthermore, it is to be understood that the respective elements illustrated in each of the embodiments may also be used in a variety of appropriate combinations.

For example, various alternative configurations not falling under the scope of the invention can be mounted on the shaft 20 instead of the distal end side surfaces 22 and 23, these alternative configurations forming examples useful for understanding the invention.

For example, as shown in FIG. 11, a groove portion 56 may be formed in the shaft 20.

This groove portion 56 is formed on the distal end surface 21 of the shaft 20 such that both ends of the groove portion 56 extend as far as edge portions of the distal end surface 21. Since the shaft 20 is formed in this manner, the suture thread S can be caught in the groove portion 56, and thereby the position of the suture thread S in the distal end surface 21 can be stabilized. As a consequence, in the above-described moving step, it is possible to prevent the position of the suture thread S from shifting from the distal end surface 21 of the shaft 20.

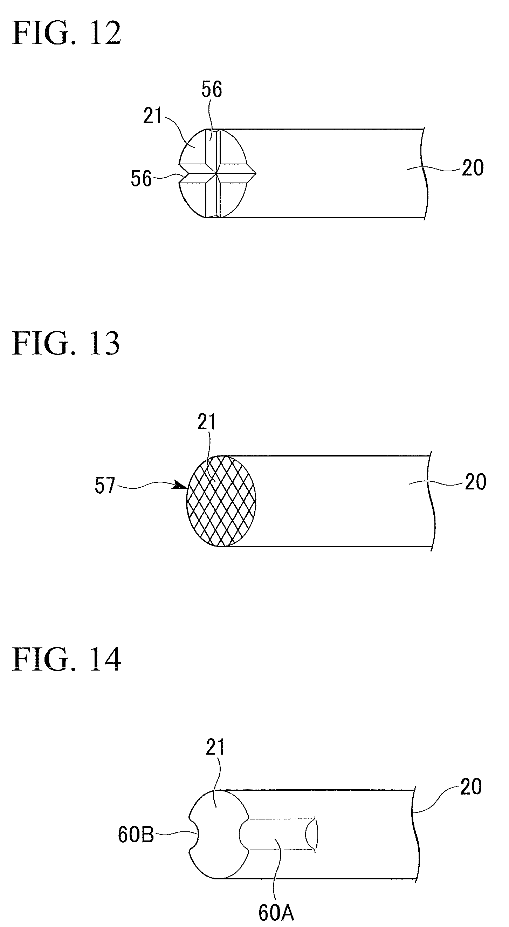

Moreover, as shown in FIG. 12, a plurality of the groove portions 56 may be formed in the distal end surface 21 of the shaft 20. In this example, two groove portions 56 that extend in directions different from each other are arranged such that they cross in the center of the distal end surface 21. In this configuration, the suture thread S can be caught in the groove portions 56 more easily.

As shown in FIG. 13, a meshed concave-convex portion 57 may be formed in the distal end surface 21 of the shaft 20. In this case as well, it is possible to prevent the position of the suture thread S from shifting from the distal end surface 21 by a friction generated between the concave-convex portion 57 and the suture thread S.

In the same way, as shown in FIG. 14, a pair of grooves 60A and 60B that extends from the distal end surface 21 of the shaft 20 may be formed in the outer surface of the shaft 20.

The grooves 60A and 60B are arranged in opposite positions on both sides of the shaft 20, and are formed so as to extend in an axial direction of the shaft 20. The grooves 60A and 60B are formed in a substantially semicircular shape in a direction parallel to the axial direction.

Since the pair of grooves 60A and 60B is formed in the shaft 20, the suture thread S can easily engage with the outer surface of the shaft 20. Furthermore, because of the grooves 60A and 60B being formed in a substantially semicircular shape, the suture thread S engaging with the grooves 60A and 60B can be easily slid in the axial direction of the shaft 20.

Note that in this modified shaft, three or more grooves may be formed.

Moreover, in the above-described pushing apparatus and pushing system, a rectangular column with the shape of a regular polygon having an even number of sides such as a square, a regular hexagon, or a regular hexacontaoctagon may be formed in the distal end of the shaft 20. In this case, because pairs of thread catch portions are formed in parallel with each other on the distal end surface of the shaft in the same way as above-described, the similar effects as the above-described can be obtained.

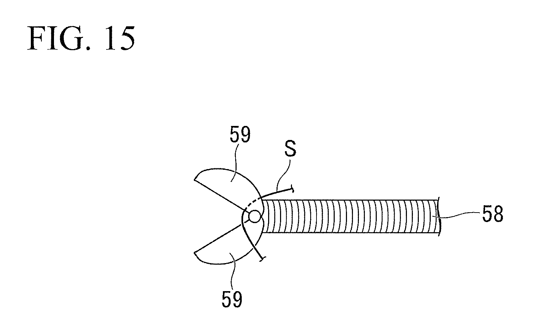

A grasping forceps 58 shown in FIG. 15 may be used in place of the shaft 20. In the above-described moving step, a pair of grasping pieces 59 is placed in an open state, and the grasping forceps 58 is pushed towards the distal side. Then, the suture thread S is sandwiched between the grasping pieces 59, and it becomes possible to more reliably prevent the position of the suture thread S from shifting away from the grasping forceps 58.

In the above-described pushing apparatus and pushing system, the slip 13 is formed as an engaging portion. However, the shape of the engaging portion is not limited to this. Provided that it is able to engage with the projection 24 on the shaft 20, the shape of the engaging portion may be a recessed portion or a protruding portion that is formed on the inner surface on the distal side of the outer cylinder 10.

Moreover, in the above-described pushing apparatus and pushing system, in the suture thread pushing method, the twist portion K is formed as a connection portion in the suture thread S. However, a knot in which the one end portion S1 of the suture thread S is able to slide relative to the other end portion S2 thereof may be formed as the connection portion.

Besides the above, the present invention is not limited by the above description, but by the appended claims.

### Industrial Applicability

According to the above-described suture thread pushing apparatus, it is possible to prevent orientations of the distal end of the shaft from shifting in the circumferential direction relative to the outer cylinder.

### Brief Description of the Referenced Symbols

- 1:: suture thread pushing apparatus
- 10:: outer cylinder
- 11, 12:: side hole
- 13:: slit (engaging portion)
- 20:: shaft (rod-shaped object)
- 22:: first distal end side surface
- 23:: second distal end side surface
- 24:: projection (engaged portion)
- 31:: suture thread pushing system
- 41:: endoscope
- 42:: insertion portion
- 43:: light-emitting unit
- 44:: light-receiving unit
- 45:: manipulation portion
- 46:: knob
- 47:: channel
- 48:: forceps opening
- 53:: guide tube
- 56:: groove portion
- 57:: concave-convex portion
- 58:: grasping forceps
- 59:: grasping piece 59
- 60A, 60B:: groove
- S:: suture thread
- K:: twist portion

## Claims

1. A suture thread pushing apparatus (1) comprising:
an outer cylinder (10) which includes a distal end portion, a proximal end portion, an internal space extending from the distal end portion to the proximal end portion, and a pair of side holes (11, 12) formed in side surfaces of the distal end portion such that a suture thread (S) is capable of being inserted through the side holes (11, 12);
a shaft member (20) which includes a distal end surface (21) which is capable of coming into contact with a knot tied in the suture thread (S) inserted through the pair of side holes (11, 12), and distal end side surfaces (22, 23), wherein gaps through which the suture thread (5) is capable of being inserted are formed between the distal end side surfaces (22, 23) and an inner surface of the outer cylinder (10) on a distal side of the pair of side holes (11, 12), the shaft member (20) being arranged so as to freely move inside the distal end portion of the outer cylinder (10) relative to the outer cylinder (10) such that the shaft member (20) pushes the knot;
an engaging portion (13) extending in an axial direction of the outer cylinder (10), the engaging portion (13) being formed in the inner surface on a distal side of the outer cylinder (10); and
an engaged portion (24) mounted on a distal side of the shaft member (20), the engaged portion (24) being configured to freely move along the engaging portion (13) and to allow the positional relationship in a circumferential direction of the outer cylinder (10) between the shaft member (20) and the outer cylinder (10) to be maintained by the engaged portion (24) engaging with the engaging portion (13) in the circumferential direction, wherein
the distal end side surfaces (22, 23) of the shaft member (20) include:
a first distal end side surface (22) formed on an outer surface of a distal end portion of the shaft member (20) and extending as far as the distal end surface (21) of the shaft member (20); and
a second distal end side surface (23) formed on the outer surface of the distal end portion of the shaft member (20) and extending as far as the distal end surface (21) of the shaft member (20), the second distal end side surface (23) being arranged on an opposite side to the first distal end side surface (22) with respect to an axis (C1) of the shaft member (20) and substantially parallel to the axis (C1) of the shaft member (20),
a proximal end of the first distal end side surface (22) is connected to a first tapered surface (22a) formed to be gradually separated from the axis (C1) of the shaft member (20) as the first tapered surface (22a) approaches the distal side of the shaft member (20), and a proximal end of the second distal end side surface (23) is connected to a second tapered surface (23a) formed to be axisymmetric to the first tapered surface (22a) about the axis (C1) of the shaft member (20), and
a width of the distal end surface (21) of the shaft member (20) is formed narrower than an outer diameter of the shaft member (20) due to the first tapered surface (22a) being connected to the first distal end side surface (22) and the second tapered surface (23a) being connected to the second distal end side surface (23).

2. The suture thread pushing apparatus according to claim 1, wherein
the engaging portion (13) is a slit formed in the outer cylinder (10), and
the engaged portion (24) is a projection protruding from an outer surface of the shaft member (20).

3. The suture thread pushing apparatus according to claim 1, wherein
spaces are formed between the outer surface of the distal end portion of the shaft member (20) and the inner surface of the outer cylinder (10) such that the suture thread (S) is kept in the spaces when the shaft member (20) is inserted into the outer cylinder and/or wherein
the shaft member (20) is formed longer than the outer cylinder (10).

4. The suture thread pushing apparatus according to claim 1, wherein
a first side hole (11) of the pair of side holes (11, 12) is configured to communicate with a first space formed between the first distal end side surface (22) and the inner surface of the outer cylinder (10) and is disposed so as to face the first distal end side surface (22), and
a second side hole (12) of the pair of side holes (11, 12) is configured to communicate with a second space formed between the second distal end side surface (23) and the inner surface of the outer cylinder (10) and is disposed so as to face the second distal end side surface (23).

5. A suture thread pushing system comprising:
the suture thread pushing apparatus (1) according to claim 1; and
an endoscope (41) including an insertion portion (42) configured to observe a distal side of the insertion portion (42).

6. The suture thread pushing system according to claim 5, further comprising
a guide tube (53) having flexibility, wherein the outer cylinder (10) is inserted into a channel of the guide tube (53) so as to advance and retreat, and the guide tube (53) is arranged in parallel with the insertion portion (42) and is fixed onto an outer surface of the insertion portion (42).

7. The suture thread pushing system according to claim 5, wherein
a channel (47) into which the outer cylinder (10) is inserted so as to advance and retreat is formed in the insertion portion (42).

8. The suture thread pushing system according to claim 5, wherein
the insertion portion (42) is inserted into a channel of the outer cylinder (10) so as to advance and retreat.

9. The suture thread pushing system according to claim 5, wherein
the outer cylinder (10) is fixed to a distal end of the insertion portion (42).

10. The suture thread pushing system according to claim 8 or 9, wherein
the insertion portion (42) is flexible, and a channel (47) through which the shaft member (20) is inserted so as to advance and retreat is formed in the insertion portion (42).

11. The suture thread pushing system according to claim 10, wherein
the shaft member (20) is formed longer than the channel (47) in the insertion portion (42).

## Patentansprüche

1. Nähfadenschiebegerät (1), das umfasst:
einen äußeren Zylinder (10), der einen distalen Endabschnitt, einen proximalen Endabschnitt, einen sich von dem distalen Endabschnitt zu dem proximalen Endabschnitt erstreckenden inneren Raum und ein Paar von Seitenöffnungen (11, 12) umfasst, die in Seitenflächen des distalen Endabschnitts ausgebildet sind, so dass ein Nähfaden (S) durch die Seitenöffnungen (11, 12) eingeführt werden kann;
ein Achselement (20), das eine distale Endfläche (21), die dazu in der Lage ist, mit einem Knoten in Kontakt zu kommen, der in den durch das Paar von Seitenöffnungen (11, 12) eingeführten Nähfaden (S) geknotet ist, und distale Endseitenflächen (22, 23) umfasst, wobei Spalte, durch die der Nähfaden (S) eingeführt werden kann, zwischen den distalen Endseitenflächen (22, 23) und einer Innenfläche des äußeren Zylinders (10) an einer distalen Seite des Paars von Seitenöffnungen (11, 12) ausgebildet sind, wobei das Achselement (20) so angeordnet ist, dass es sich frei im Inneren des distalen Endabschnitts des äußeren Zylinders (10) relativ zu dem äußeren Zylinder (10) bewegt, so dass das Achselement (20) den Knoten schiebt;
einen Eingriffsabschnitt (13), der sich in einer axialen Richtung des äußeren Zylinders (10) erstreckt, wobei der Eingriffsabschnitt (13) in der Innenfläche auf einer distalen Seite des äußeren Zylinders (10) ausgebildet ist; und
einen in Eingriff stehenden Abschnitt (24), der auf einer distalen Seite des Achselements (20) befestigt ist, wobei der in Eingriff stehende Abschnitt (24) dazu eingerichtet ist, sich frei entlang des Eingriffsabschnitts (13) zu bewegen und es ermöglicht, die Positionsbeziehung in einer Umfangsrichtung des äußeren Zylinders (10) zwischen dem Achselement (20) und dem äußeren Zylinder (10) beizubehalten, indem der in Eingriff stehende Abschnitt (24) in der Umfangsrichtung mit dem Eingriffsabschnitt (13) in Eingriff steht, wobei
die distalen Endseitenflächen (22, 23) des Achselements (20) umfassen:
eine erste distale Endseitenfläche (22), die an einer Außenfläche eines distalen Endabschnitts des Achselements (20) ausgebildet ist und sich so weit erstreckt wie die distale Endfläche (21) des Achselements (20); und
eine zweite distale Endseitenfläche (23), die an der Außenfläche des distalen Endabschnitts des Achselements (20) ausgebildet ist und sich so weit erstreckt wie die distale Endfläche (21) des Achselements (20), wobei die zweite distale Endseitenfläche (23) bezüglich einer Achse (C1) des Achselements (20) auf einer entgegengesetzten Seite zu der ersten distalen Endseitenfläche (22) und im Wesentlichen parallel zu der Achse (C1) des Achselements (20) angeordnet ist,
ein proximales Ende der ersten distalen Endseitenfläche (22) mit einer ersten geneigten Oberfläche (22a) verbunden ist, die so ausgebildet ist, dass sie sich graduell von der Achse (C1) des Achselements (20) löst, wenn sich die erste geneigte Oberfläche (22a) der distalen Seite des Achselements (20) annähert, und ein proximales Ende der zweiten distalen Endseitenfläche (23) mit einer zweiten geneigten Oberfläche (23a) verbunden ist, die so ausgebildet ist, dass sie asymmetrisch zu der ersten geneigten Oberfläche (22a) um die Achse (C1) des Achselements (20) ist, und
eine Breite der distalen Endfläche (21) des Achselements (20) schmaler ausgebildet ist als ein Außendurchmesser des Achselements (20), da die erste geneigte Oberfläche (22a) mit der ersten distalen Endseitenfläche (22) verbunden ist und die zweite geneigte Oberfläche (23a) mit der zweiten distalen Endseitenfläche (23) verbunden ist.

2. Nähfadenschiebegerät gemäß Anspruch 1, wobei
der Eingriffsabschnitt (13) ein in dem äußeren Zylinder (10) ausgebildeter Schlitz ist und
der in Eingriff stehende Abschnitt (24) ein Vorsprung ist, der von einer Außenfläche des Achselements (20) vorsteht.

3. Nähfadenschiebegerät gemäß Anspruch 1, wobei
Spalte zwischen der Außenfläche des distalen Endabschnitts des Achselements (20) und der Innenfläche des äußeren Zylinders (10) derart ausgebildet sind, dass der Nähfaden (S) in den Spalten gehalten wird, wenn das Achselement (20) in den äußeren Zylinder eingeführt wird, und/oder wobei
das Achselement (20) länger ausgebildet ist als der äußere Zylinder (10).

4. Nähfadenschiebegerät gemäß Anspruch 1, wobei
eine erste Seitenöffnung (11) des Paars von Seitenöffnungen (11, 12) dazu eingerichtet ist, mit einem ersten Spalt zu kommunizieren, der zwischen der ersten distalen Endseitenfläche (22) und der Innenfläche des äußeren Zylinders (10) ausgebildet ist, und so angeordnet ist, dass sie der ersten distalen Endseitenfläche (22) zugewandt ist, und
eine zweite Seitenöffnung (12) des Paars von Seitenöffnungen (11, 12) dazu eingerichtet ist, mit einem zweiten Spalt zu kommunizieren, der zwischen der zweiten distalen Endseitenfläche (23) und der Innenfläche des äußeren Zylinders (10) ausgebildet ist, und so angeordnet ist, dass sie der zweiten distalen Endseitenfläche (23) zugewandt ist.

5. Nähfadenschiebesystem, das umfasst:
das Nähfadenschiebegerät (1) gemäß Anspruch 1; und
ein Endoskop (41), das einen Einführabschnitt (42) umfasst, der dazu eingerichtet ist, eine distale Seite des Einführabschnitts (42) zu beobachten.

6. Nähfadenschiebesystem gemäß Anspruch 5, das ferner umfasst:
ein Führungsrohr (53), das Flexibilität aufweist, wobei der äußere Zylinder (10) in einen Kanal des Führungsrohrs (53) eingeführt ist, um sich vorwärts und rückwärts zu bewegen, und das Führungsrohr (53) parallel zu dem Einführabschnitt (42) angeordnet ist und an einer Außenfläche des Einführabschnitts (42) befestigt ist.

7. Nähfadenschiebesystem gemäß Anspruch 5, wobei
ein Kanal (47), in den der äußere Zylinder (10) eingeführt ist, um sich vorwärts und rückwärts zu bewegen, in dem Einführabschnitt (42) ausgebildet ist.

8. Nähfadenschiebesystem gemäß Anspruch 5, wobei
der Einführabschnitt (42) in einen Kanal des äußeren Zylinders (10) eingeführt ist, um sich vorwärts und rückwärts zu bewegen.

9. Nähfadenschiebesystem gemäß Anspruch 5, wobei
der äußere Zylinder (10) an einem distalen Ende des Einführabschnitts (42) befestigt ist.

10. Nähfadenschiebesystem gemäß Anspruch 8 oder 9, wobei
der Einführabschnitt (42) flexibel ist und ein Kanal (47), durch den das Achselement (20) eingeführt ist, um sich vorwärts und rückwärts zu bewegen, in dem Einführabschnitt (42) ausgebildet ist.

11. Nähfadenschiebesystem gemäß Anspruch 10, wobei
das Achselement (20) länger ausgebildet ist als der Kanal (47) in dem Einführabschnitt (42).

## Revendications

1. Appareil (1) de poussée de fil de suture comprenant :
un cylindre extérieur (10) qui inclut une partie d'extrémité distale, une partie d'extrémité proximale, un espace interne s'étendant de la partie d'extrémité distale jusqu'à la partie d'extrémité proximale, et une paire de trous latéraux (11, 12) formés dans des surfaces latérales de la partie d'extrémité distale de telle sorte qu'un fil de suture (S) est apte à être inséré à travers les trous latéraux (11, 12) ;
un élément (20) d'arbre qui inclut une surface (21) d'extrémité distale qui est apte à venir en contact avec un noeud lié dans le fil de suture (S) inséré à travers la paire de trous latéraux (11, 12), et des surfaces latérales (22, 23) d'extrémité distale, dans lequel des espaces à travers lesquels le fil de suture (S) est apte à être inséré sont formés entre les surfaces latérales (22, 23) d'extrémité distale et une surface intérieure du cylindre extérieur (10) sur un côté distal de la paire de trous latéraux (11, 12), l'élément (20) d'arbre étant agencé de façon à se déplacer librement à l'intérieur de la partie d'extrémité distale du cylindre extérieur (10) par rapport au cylindre extérieur (10) de telle sorte que l'élément (20) d'arbre pousse le noeud ;
une partie (13) d'engagement s'étendant dans un sens axial du cylindre extérieur (10), la partie (13) d'engagement étant formée dans la surface intérieure sur un côté distal du cylindre extérieur (10) ; et
une partie (24) engagée montée sur un côté distal de l'élément (20) d'arbre, la partie (24) engagée étant configurée pour se déplacer librement le long de la partie (13) d'engagement et pour permettre que la relation positionnelle dans un sens circonférentiel du cylindre extérieur (10) entre l'élément (20) d'arbre et le cylindre extérieur (10) soit maintenue par la partie (24) engagée s'engageant avec la partie (13) d'engagement dans le sens circonférentiel, dans lequel
les surfaces latérales (22, 23) d'extrémité distale de l'élément (20) d'arbre incluent :
une première surface latérale (22) d'extrémité distale formée sur une surface extérieure d'une partie d'extrémité distale de l'élément (20) d'arbre et s'étendant aussi loin que la surface (21) d'extrémité distale de l'élément (20) d'arbre ; et
une deuxième surface latérale (23) d'extrémité distale formée sur une surface extérieure de la partie d'extrémité distale de l'élément (20) d'arbre et s'étendant aussi loin que la surface (21) d'extrémité distale de l'élément (20) d'arbre, la deuxième surface latérale (23) d'extrémité distale étant agencée sur un côté opposé à la première surface latérale (22) d'extrémité distale par rapport à un axe (C1) de l'élément (20) d'arbre et sensiblement parallèle à l'axe (C1) de l'élément (20) d'arbre,
une extrémité proximale de la première surface latérale (22) d'extrémité distale est connectée à une première surface (22a) conique formée pour être graduellement séparée de l'axe (C1) de l'élément (20) d'arbre alors que la première surface (22a) conique approche de l'extrémité distale de l'élément (20) d'arbre, et une extrémité proximale de la deuxième surface latérale (23) d'extrémité distale est connectée à une deuxième surface (23a) conique formée pour être axisymétrique à la première surface (22a) conique autour de l'axe (C1) de l'élément (20) d'arbre, et
une largeur de la surface (21) d'extrémité distale de l'élément (20) d'arbre est formée plus étroite qu'un diamètre extérieur de l'élément (20) d'arbre du fait de la première surface (22a) conique étant connectée à la première surface latérale (22) d'extrémité distale et de la deuxième surface (23a) conique étant connectée à la deuxième surface latérale (23) d'extrémité distale.

2. Appareil de poussée de fil de suture selon la revendication 1, dans lequel
la partie (13) d'engagement est une fente formée dans le cylindre extérieur (10), et
la partie (24) engagée est une projection se projetant depuis une surface extérieure de l'élément (20) d'arbre.

3. Appareil de poussée de fil de suture selon la revendication 1, dans lequel
des espaces sont formés entre la surface extérieure de la partie d'extrémité distale de l'élément (20) d'arbre et la surface intérieure du cylindre extérieur (10) de telle sorte que le fil de suture (S) est maintenu dans les espaces lorsque l'élément (20) d'arbre est inséré dans le cylindre extérieur et/ou dans lequel
l'élément (20) d'arbre est formé plus long que le cylindre extérieur (10).

4. Appareil de poussée de fil de suture selon la revendication 1, dans lequel
un premier trou latéral (11) de la paire de trous latéraux (11, 12) est configuré pour communiquer avec un premier espace formé entre la première surface latérale (22) d'extrémité distale et la surface intérieure du cylindre extérieur (10) et est disposé de façon à faire face à la première surface latérale (22) d'extrémité distale, et
un deuxième trou latéral (12) de la paire de trous latéraux (11, 12) est configuré pour communiquer avec un deuxième espace formé entre la deuxième surface latérale (23) d'extrémité distale et la surface intérieure du cylindre extérieur (10) et est disposé de façon à faire face à la deuxième surface latérale (23) d'extrémité distale.

5. Système de poussée de fil de suture comprenant :
l'appareil (1) de poussée de fil de suture selon la revendication 1 ; et
un endoscope (41) incluant une partie (42) d'insertion configurée pour observer un côté distal de la partie (42) d'insertion.

6. Système de poussée de fil de suture selon la revendication 5, comprenant en outre
un tube guide (53) ayant une flexibilité, dans lequel le cylindre extérieur (10) est inséré dans un canal du tube guide (53) de façon à avancer et se rétracter, et le tube guide (53) est agencé en parallèle avec la partie (42) d'insertion et est fixé sur une surface extérieure de la partie (42) d'insertion.

7. Système de poussée de fil de suture selon la revendication 5, dans lequel
un canal (47) dans lequel le cylindre extérieur (10) est inséré de façon à avancer et se rétracter est formé dans la partie (42) d'insertion.

8. Système de poussée de fil de suture selon la revendication 5, dans lequel
la partie (42) d'insertion est insérée dans un canal du cylindre extérieur (10) de façon à avancer et se rétracter.

9. Système de poussée de fil de suture selon la revendication 5, dans lequel
le cylindre extérieur (10) est fixé à une extrémité distale de la partie (42) d'insertion.

10. Système de poussée de fil de suture selon la revendication 8 ou 9, dans lequel
la partie (42) d'insertion est flexible, et un canal (47) à travers lequel l'élément (20) d'arbre est inséré de façon à avancer et se rétracter est formé dans la partie (42) d'insertion.

11. Système de poussée de fil de suture selon la revendication 10, dans lequel
l'élément (20) d'arbre est formé plus long que le canal (47) dans la partie (42) d'insertion.
